## Europäisches Patentamt

## European Patent Office

## Office européen des brevets

(11) Veröffentlichungsnummer: **0 225 964**
**B1**

(12) # EUROPÄISCHE PATENTSCHRIFT

(45) Veröffentlichungstag der Patentschrift:
25.10.89

(51) Int. Cl.⁴: **A61B 6/00**, A61B 6/03,
F25D 17/02

(21) Anmeldenummer: 86109895.2

(22) Anmeldetag: 18.07.86

(54) Vorrichtung zum Kühlen von Komponenten einer Röntgenanlage.

(30) Priorität: 01.08.85 DE 3527657

(43) Veröffentlichungstag der Anmeldung:
24.06.87 Patentblatt 87/26

(45) Bekanntmachung des Hinweises auf die Patenterteilung:
25.10.89 Patentblatt 89/43

(84) Benannte Vertragsstaaten:
DE FR GB

(56) Entgegenhaltungen:
EP-A- 0 081 751
EP-A- 0 109 206
EP-A- 0 182 040
GB-A- 2 026 812
GB-A- 2 034 149

IEEE TRANSACTIONS ON NUCLEAR SCIENCE, Band
NS-26, Nr. 2, Teil II, April 1979, Seiten 2836-2839, IEEE,
New York, US; D.P. BOYD: "Status of diagnostic X-ray
CT: 1979"
PATENTS ABSTRACTS OF JAPAN, Band 6,
Nr. 125 (P-127)[1003], 10. Juli 1982; &
JP-A-57 50 673 (TOKYO SHIBAURA DENKI
K.K.) 25.03.1982

(73) Patentinhaber: Siemens Aktiengesellschaft,
Wittelsbacherplatz 2, D-8000 München 2(DE)

(72) Erfinder: Dotzauer, Peter, Dipl.-Ing., Eisenstrasse 15,
D-8520 Buckenhof(DE)
Erfinder: Schmidt, Martin, Dipl.-Ing., Eckartstrasse 9,
D-8535 Emskirchen(DE)

**Beschreibung**

Die Erfindung betrifft eine Vorrichtung zum Kühlen von Anlagenkomponenten auf einem Rotor, der in einem feststehenden Geräteteil drehbar gelagert ist.

Aus EP-A1 0 182 040 (veröffentlicht am 28.05.86) ist eine solche Vorrichtung bekannt.

Diese Vorrichtung weist einen abgedichteten Kanal auf, welcher zwischen dem feststehenden Geräteteil und dem Rotor angeordnet ist.

Gemäß Artikel 54(3) EPÜ gilt der Inhalt dieser europäischen Patentanmeldung als Stand der Technik für die Staaten DE, FR.

Bei einem in der Medizintechnik zur Erzeugung von Schichtbildern eines Patienten verwendeten Computertomographen muß die Verlustwärme des Strahlendetektors und/oder der Meßelektronik abgeführt werden. Hierzu kann ein Ölkreislauf vorgesehen werden, in dem Kühlöl zwischen der zu kühlenden Komponente und einem durch ein Gebläse gekühlten Ölkühler zirkuliert. Der Ölkühler muß dabei auf dem Rotor angeordnet werden, wodurch eine starke Erwärmung des Innenraumes des Computertomographen bedingt ist. Außerdem können sich Wärmenester ausbilden. Dadurch wird in starkem Maße die empfindliche Meßelektronik negativ beeinflußt.

Der Erfindung liegt die Aufgabe zugrunde, eine Vorrichtung der eingangs genannten Art so auszubilden, daß eine gezielte Abführung der Verlustwärme vom Rotor nach außen erfolgt.

Diese Aufgabe ist erfindungsgemäß dadurch gelöst, daß zwischen dem feststehenden Geräteteil und dem Rotor ein abgedichteter Kanal vorgesehen ist, in dem der auf der Innenseite des feststehenden Geräteteils (1, 2) befestigte Verdampfer eines Kühlgerätes liegt, und daß der Kanal mit einer Kühlflüssigkeit eines auf dem Rotor vorgesehenen geschlossenen Kühlkreises gefüllt ist, welcher die zu kühlende Komponente und eine Kühlflüssigkeitspumpe enthält. Bei der erfindungsgemäßen Vorrichtung wird die auf dem Rotor entstehende Verlustwärme mit Hilfe einer Kühlflüssigkeit in den Kanal zwischen feststehendem Geräteteil und Rotor gepumpt und dort durch ein Kühlgerät nach außen befördert, dessen Verdampfer in diesem Kanal angeordnet ist. Die Erfindung eignet sich insbesondere bei einem Computertomographen zur Kühlung des rotierenden Strahlendetektors. Das Kühlgerät kann dabei getrennt vom Computertomographen aufgestellt werden, so daß eine gezielte Wärmeabführung in einen Bereich, in dem sie nicht mehr stört, möglich ist.

Die Erfindung ist nachfolgend anhand eines in der Zeichnung dargestellten Ausführungsbeispieles in Verbindung mit einem Computertomographen näher erläutert. Es zeigen:

Fig. 1 die für die Erfindung wesentlichen Teile eines Computertomographen,

Fig. 2 eine teilweise geschnittene Seitenansicht des Computertomographen nach Figur 1,

Fig. 3 einen Schnitt nach der Linie III-III in Figur 1, und

Fig. 4 eine Prinzipdarstellung zur Erläuterung des Kühlkreislaufes des Computertomographen gemäß den Figuren 1 bis 3.

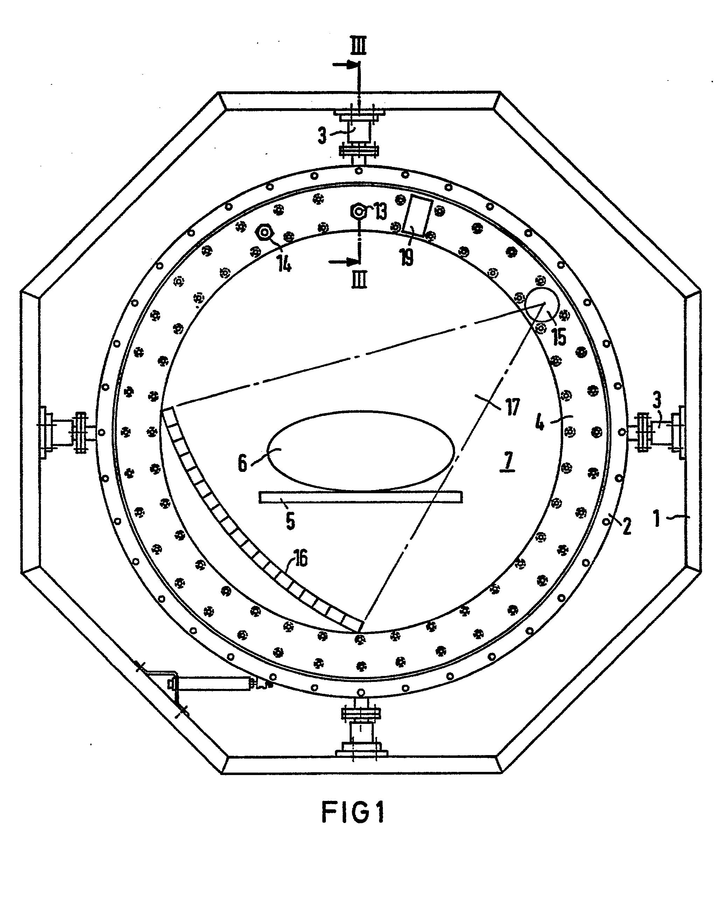

In der Figur 1 ist ein Rahmen 1 eines Computertomographen dargestellt, der zusammen mit einem Ring 2 einen feststehenden Geräteteil bildet. Der Ring 2 ist dabei durch Stützen 3 im Rahmen 1 abgestützt. Um den Ring 2 rotiert ein Rotor 4, mit dem ein Röntgenstrahler 15 und ein Strahlendetektor 16 fest verbunden ist. Der Röntgenstrahler 15 sendet ein fächerförmiges Röntgenstrahlenbündel 17 aus, das einen auf einer Liege 5 liegenden Patienten 6 in einem Untersuchungsraum 7 durchstrahlt. Der Strahlendetektor 16 besteht dabei aus einer Reihe von Detektorelementen, deren Ausgangssignale, die bei der Drehung des Rotors 4 mit dem Röntgenstrahler 15 und dem Strahlendetektor 16 um 360° um den Patienten 6 gebildet werden, einem Rechner zugeführt werden, der daraus ein Transversalschichtbild des Patienten 6 berechnet.

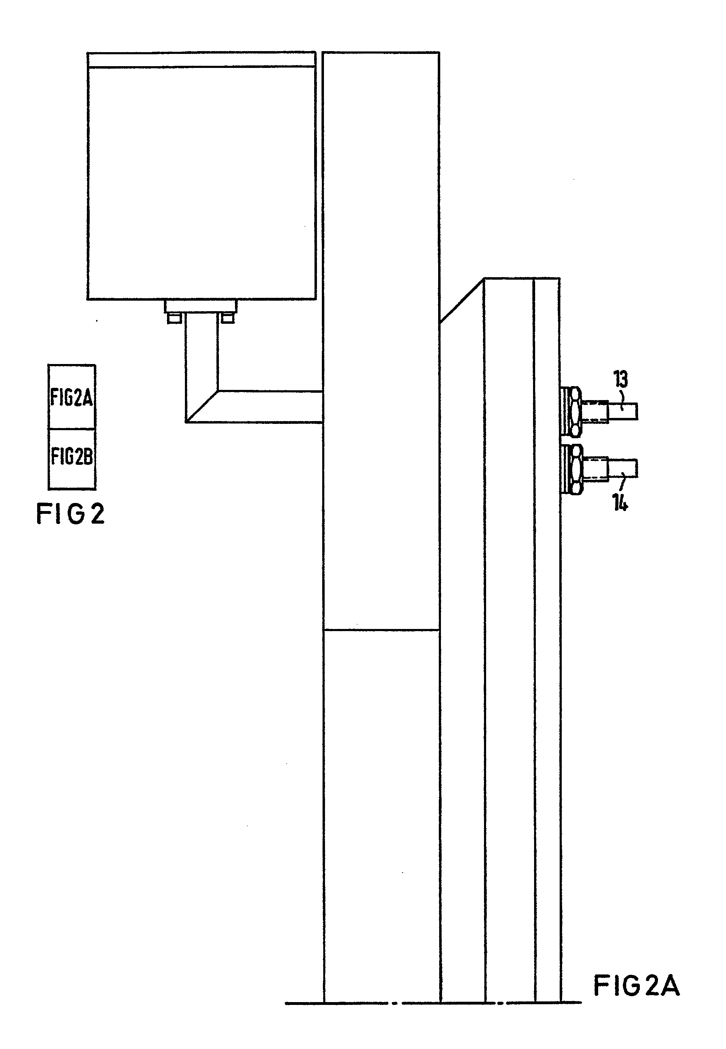

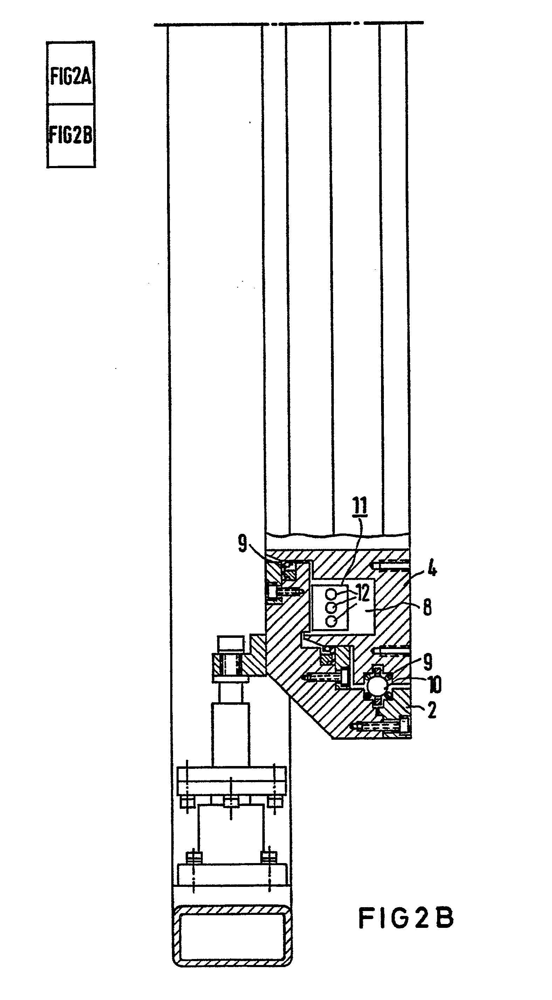

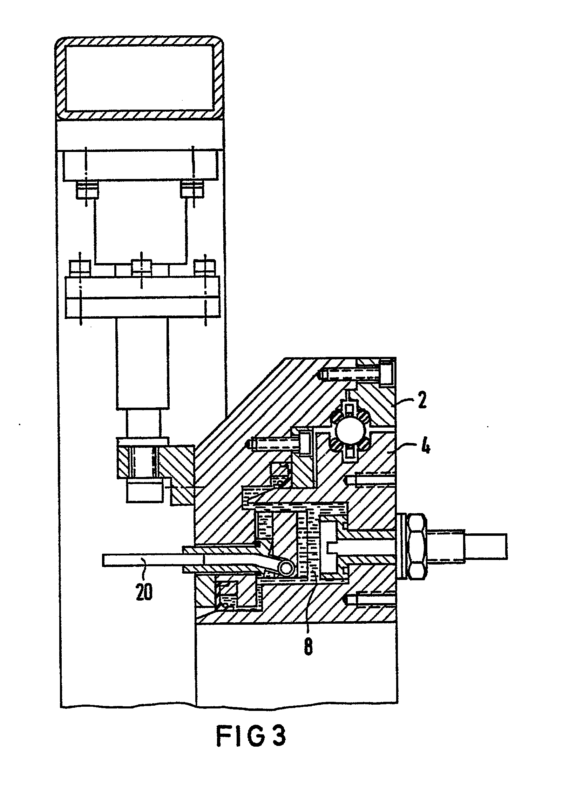

Insbesondere bei Computertomographen mit Dauerrotation des Rotors zur Erzielung kurzer Abtastzeiten ist es erforderlich, den Strahlendetektor zu kühlen. Hierzu ist zwischen dem Ring 2 und dem Rotor 4 ein Kanal 8 (Figuren 2, 3) vorgesehen, der durch Dichtringe 9 nach außen abgedichtet ist. Ein Lager 10 ermöglicht dabei eine Rotation des Rotors 4 gegenüber dem Ring 2. Im Kanal 8 ist ein Verdampfer 11 eines Kühlgerätes angeordnet, von dem in der Figur 2 drei Kanäle 12 für das Kühlmedium sichtbar sind. Der Verdampfer 11 ist auf der Innenseite des Ringes 2 befestigt. Das Kühlgerät kann dabei stationär neben dem Computertomographen angeordnet und durch Leitungen mit dem Verdampfer 12 verbunden sein.

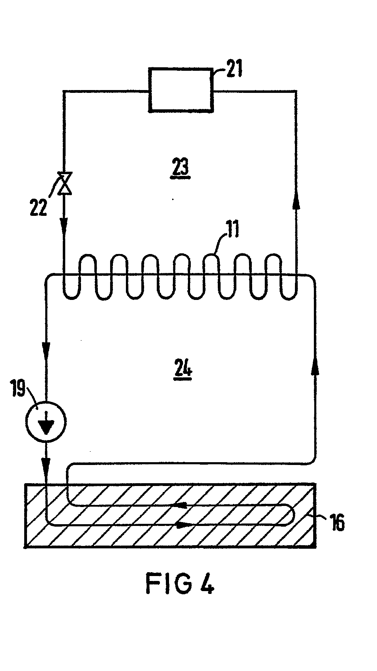

Der Kanal 8 ist mit einer Kühlflüssigkeit, z.B. Alkohol, Öl usw. gefüllt, die in einem geschlossenen Kühlkreis auf dem Rotor 4 mit Hilfe einer Pumpe 19 (Fig. 4) zum Strahlendetektor 16 und von diesem wieder zurückgeführt wird. In den Figuren 1, 2 sind zwei Anschlüsse 13, 14 gezeigt, die die Hin- bzw. Rückleitung für die Kühlflüssigkeit bilden. Durch die Kühlflüssigkeit wird demgemäß die am Strahlendetektor 16 entstehende Wärme zum Verdampfer 11 geführt. Das Kühlgerät pumpt dabei die Wärme an eine Stelle, wo sie nicht mehr stört.

Die Kanäle 12 des Verdampfers 11 sind über zwei Stutzen, von denen in der Figur 3 der Stutzen 20 sichtbar ist, an einem separaten Kühlaggregat 21 (Fig. 4) angeschlossen.

Die Figur 4 zeigt, das das Kühlaggregat 21 über ein Expansionsventil 22 am Verdampfer 11 angeschlossen ist und mit diesem einen geschlossenen, feststehenden Kreislauf 23 bildet. Diesem ist ein rotierender Kreislauf 24 mit der Kühlflüssigkeitspumpe 19 und dem zu kühlenden Objekt, d.h. dem Strahlendetektor 16, zugeordnet.

Bei dem dargestellten Kühlsystem ergeben sich folgende Vorteile:

1. Es besteht die Möglichkeit der Kühlung eines rotierenden Systems, bei dem ein Zugriff über eine Drehachse nicht möglich ist.

2. Das Kühlsystem ist innerhalb eines weiten Größenbereichen von bis zu vier Meter Durchmesser herstellbar.

3. Das Kühlaggregat 21 ist als separates Gerät über Kupplungen angeflanscht.

4. Es besteht eine hohe Temperaturkonstanz im zu kühlenden Objekt.

5. Das System ist in einem weiten Temperaturbereich anwendbar.

**Patentansprüche**

1. Vorrichtung zum Kühlen von Anlagenkomponenten (16) auf einem Rotor (4), der in einem feststehenden Geräteteil (1, 2), drehbar gelagert ist, wobei zwischen dem feststehenden Geräteteil (1, 2) und dem Rotor (4) ein abgedichteter Kanal (8) vorgesehen ist, in dem der auf der Innenseite des feststehenden Geräteteils (1, 2) befestigte Verdampfer (11) eines Kühlgerätes (21) liegt, und der Kanal (8) mit einer Kühlflüssigkeit eines auf dem Rotor (4) vorgesehenen geschlossenen Kühlkreises (24) gefüllt ist, welcher die zu kühlende Anlagenkomponente (16) und eine Kühlflüssigkeitspumpe (19) enthält.

2. Vorrichtung nach Anspruch 1, dadurch gekennzeichnet, daß der Rotor (4) Bestandteil eines Computertomographen ist und an gegenüberliegenden Seiten eines den Patienten aufnehmenden Raumes (7) einen Röntgenstrahler (15) und einen Strahlendetektor (16) trägt, welcher im Kühlkreis (24) auf dem Rotor (4) liegt.

**Claims**

1. Arrangement for cooling equipment components (16) on a rotor (4) which is rotatably mounted in a fixed apparatus part (1, 2), there being provided between the fixed apparatus part (1, 2) and the rotor (4) a sealed channel (8) in which lies the evaporator (11) of a cooling apparatus (21), which evaporator is secured to the inside of the fixed apparatus part (1, 2), and the channel (8) being filled with a coolant of a closed cooling circuit (24) which is provided on the rotor (4) and which includes the equipment component (16) to be cooled and a coolant pump (19).

2. Arrangement according to claim 1, characterised in that the rotor (4) is part of a computer tomograph and supports on opposite sides of a chamber (7), which receives the patient, an X-ray source (15) and a ray detector (16) which lies in the cooling circuit (24) on the rotor (4).

**Revendications**

1. Dispositif pour refroidir des composants (16) d'une installation placés sur un rotor (4), qui est monté de façon à pouvoir tourner dans une partie fixe (1, 2) d'un appareil, et dans lequel entre la partie fixe (1, 2) de l'appareil et le rotor (4), il est prévu un canal étanchéifié (8), dans lequel est disposé l'évaporateur (11), fixé sur la face intérieure de la partie fixe (1, 2) de l'appareil, d'un apparail de refroidissement (21), et dans lequel le canal (8) est rempli par un liquide de refroidissement d'un circuit fermé de refroidissement prévu sur le rotor (4) et contenant les composants (16) de l'installation à refroidir et une pompe (19) d'entraînement du liquide de refroidissement.

2. Dispositif suivant la revendication 1, caractérisé par le fait que le rotor (4) fait partie d'un appareil de tomodensitométrie et porte, sur des côtés opposés d'un espace (7) recevant le patient, un émetteur radiologique (4) et un détecteur de rayonnement (16), qui est situé dans le circuit de refroidissement (24) installé sur le rotor (4).

FIG 1

FIG 2

FIG2A

FIG2B

FIG 2A

13

14

FIG2A

FIG2B

FIG 2 B

FIG 3

FIG 4